# EUROPEAN PATENT APPLICATION

(11) **EP 2 698 141 A1**
(43) Date of publication of application: **19.02.2014**
(21) Application number: 12180433.0
(22) Date of filing: 14.08.2012
(51) Int. Cl.: A61H 31/00

(54) **Apparatus for assisting cardiopulmonary resuscitation**

(71) Applicant: Schiller AG, 6341 Baar (CH)
(72) Inventor: Fontaine, Guy, 94160 Saint Mandé (FR); Schmid, Johann-Jakob, 8911 Rifferswil (CH)
(74) Representative: Hepp Wenger Ryffel AG

(57) **Abstract**

The invention relates to an apparatus (1) for assisting cardiopulmonary resuscitation by applying compressions to the chest of a patient to stimulate blood circulation. The apparatus (1) comprises a pneumatic compressor assembly (10) including at least an actuator (1) and an application member (20) for pressing against an application site of the patient. The pneumatic compressor assembly (10) is connected or connectable to a pressurized gas container (70) by a connecting member (50) while the connecting member (50) is adapted for the operation of the pneumatic compressor assembly (10) with a gas or mixture of gas in liquid form.

## Description

The present invention relates to an apparatus for assisting cardiopulmonary resuscitation by applying compressions to the chest of a patient to stimulate blood circulation. In particular, the apparatus of the present invention comprises a pneumatic compressor for applying chest compressions.

Such artificial stimulation of the blood circulation is particularly indicated for a person suffering from a cardiovascular emergency such as cardiac arrest. Applying chest compressions is part of cardiopulmonary resuscitation (CPR) as an emergency procedure.

Apparatus for applying compressions to the chest of a patient by a pneumatic compressor are known in the prior art.

US 7,060,041 B1 discloses a pneumatic apparatus for applying compressions to the chest of a patient to stimulate blood circulation comprising an actuator which is energized by pressurized gas. The pressurized gas is provided from a pressured gas container. An adjuster varies the gas pressure applied to the apparatus.

Such apparatus has the disadvantage that there is need for a large-scale pressurized gas container or on the other hand, the pressurized gas container needs to be exchanged frequently. A further disadvantage is the necessity to adjust in a complex way the gas pressure supplied to the device for applying compressions to the chest of a patient.

Another known apparatus with a pneumatic compressor is the "Weil™ Mini Chest Compressor" for assisting cardiopulmonary resuscitation. The gas source used for the operation of such apparatus is pressurized air or pressurized oxygen (O₂). The consumption of pressurized gas is about 60 litres per minute during adequate operation of such apparatus for cardiopulmonary resuscitation.

Such apparatus has the disadvantage that for a long autonomous operation, i.e. energized only by the stored gas in the container, a large-scale and heavy-weight pressurized gas container is required.

It is therefore an object of the present invention to solve the problems of the prior art. It is particularly an object to provide a simplified light weight apparatus with a pneumatic compressor for applying compressions to the chest of a patient which does not require frequent replacement of the gas source.

This object is solved by an apparatus according to the characterizing portions of the independent claim.

The apparatus according to the present invention for assisting cardiopulmonary resuscitation by applying compressions to the chest of a patient to stimulate blood circulation comprises a pneumatic compressor assembly including an actuator and an application member for pressing against an application site of the patient. In principle, such pneumatic compressor assembly is comparable to the "Weil™ Mini Chest Compressor" as known from the prior art.

The application site may typically be the sternum of the patient. Other sites might also be conceivable.

Preferably, the actuator is functionally connected to the application member. More preferably, the application member comprises a pressing member for pressing against the application site which is functionally connected to the actuator. The pressing member preferably has a substantially round profile with a diameter of preferably 4 to 10 cm, more preferably 5 to 9 cm, most preferably 6 to 8 cm.

Preferably, the application member is fixable at the patient's torso by a fixing means. The fixing means may be a belt.

Alternatively, the application member may be configured to be hold manually to the application site of the patient by a rescuer.

The pneumatic compressor assembly is connected or connectable to a pressurized gas container by a connecting member.

The connecting member is adapted for the operation of the pneumatic compressor assembly with a pressurized gas or mixture of gas stored in liquid form. The connecting member may be designed as a direct connection unit between the pressurized gas container and the pneumatic compressor assembly comprising connecting means allowing connecting and disconnecting of the pressurized gas container, e.g. for replacement of empty gas containers. The connecting means may comprise a screw thread. Preferably, the connecting member can be placed directly at the pressurized gas container and connected to the pneumatic compressor assembly by means of a flexible tube. More preferably, the connecting member is provided with an opening and closing mechanism.

It has been found that such an apparatus for applying compressions to the chest of a patient is simplified.

The pressure of gas stored in gaseous state for the operation of the prior art devices has to be around 300 bar (i.e. around 4500 psi), while e.g. liquefied carbon dioxide can be stored at a pressure of around 60 bar. The connecting member can therefore be made of a slight material and without the need of any reinforcement means. The size of the connecting member can further be reduced. Since the connecting member has to sustain a lower pressure of around 60 bar, its design can be simplified.

By using a gas or mixture of gas in liquid form the size of a pressurized gas container can advantageously be reduced. Moreover, since a gas or mixture of gases in liquid form can be stored at lower pressure than a gas in gaseous state, the pressurized gas container can be made of a slight composite material.

In a preferred embodiment, the pressurized gas container is part of the apparatus and contains a gas or a mixture of gas stored in liquid form.

The vapour pressure established in the ullage of a pressurized gas container containing gas in liquid form remains constant independent from the fluid level in the container. A constant vapour pressure simplifies the supply of pressurized gas to the apparatus and supports constant operating conditions.

Preferably, the pressurized gas container has a quantity size of 1 to 5 l.

A gas or mixture of gas stored in liquid form means that at least one gas is in essentially liquid form.

A gas is usually brought in liquid form by cooling and / or compressing it. Therefore, the storage of a gas or mixture of gas in liquid form may be guaranteed by maintaining a sufficient cooling and / or a sufficient pressure in a storage means. A p-T phase diagram may provide information regarding the necessary physical condition (pressure and temperature) for storing a certain gas in liquid form. Preferably, a gas or mixture of gas is stored in the pressurized gas container at a sufficient pressure without any need for coolant means.

Preferably, a gas or mixture of gas is used having a partial pressure at a minimum operating temperature of around 0°C which is 35 bar and thus higher than the pressure as desired for operation of the pneumatic compressor assembly.

Regarding the operation of the apparatus of the present invention, a gas or mixture of gas can preferably be stored in liquid form in the pressurized gas container at a pressure which is close to the gas pressure as desired for operation of the pneumatic compressor assembly.

Principally, any gas or mixture of gas stored in liquid form having a sufficient high partial pressure may be suitable for the operation of the pneumatic compressor assembly by the usage of a gas pressure reducing means.

Preferably, the gas source is a source of carbon dioxide.

Usage of carbon dioxide has the advantage - as can be seen in a p-T phase diagram - that stored at a pressure around 60 bar, carbon dioxide does not have to be cooled.

Moreover, it has been found that about 3kg of pressurized carbon dioxide at 60 bar stored in liquid form suffices for the adequate and autonomous operation of the apparatus of the present invention for cardiopulmonary resuscitation for 30 minutes at a gas pressure adjusted for operation of the pneumatic compressor assembly at around 4 bar.

Preferably, the apparatus is operated autonomously, i.e. the pneumatic compressor assembly is energized only by the gas stored in the liquid phase. Thus, the apparatus can easily be transported.

A cooling effect because of the change of the liquid to a gaseous state may occur at the pressurized gas container. During transportation, the pressurized gas container is usually positioned between the legs of the patient. Preferably, the pressurized gas container is provided with at least one winglet ensuring a certain distance between the patient and the container. Such winglet may avoid direct contact between the thighs of the patient and the cold pressurized gas container during transportation.

Alternatively, the pressurized gas container may comprise a contact surface for cooling. Such means may be used for cooling of the patient's body.

According to a preferred embodiment, the apparatus comprises a gas pressure reducing means between the pressurized gas container and the pneumatic compressor assembly. Preferably, the gas pressure reducing means is included in or coupled to the connecting member. More preferably, the gas pressure reducing means is located at or towards the exit of the pressurized gas container.

Preferably, the gas pressure reducing means is provided by a gas pressure regulator, preferably a membrane gas pressure regulator, adapted to regulate and / or control the applied gas pressure as desired for operation of the pneumatic compressor assembly.

Alternatively, the gas pressure reducing means may be provided by a pressure reducing valve. The typical functioning of a pressure reducing valve is based on limiting the passage of a gas flow.

Alternatively, the gas pressure reducing means may be provided by a means comprising a hole of a diameter which is smaller than the diameter at the exit of the pressurized gas container. This is a simple construction of a gas pressure reducing means.

A changing gas pressure of a gas stored in gaseous state has the disadvantage that the amount of pressure reduction has to be changed continuously during operation, contrary to a gas stored in liquid form with a constant vapour pressure which allows a pressure reduction continuously at the same rate.

The pressure reducing valve may be adapted to regulate and / or control the applied gas pressure as desired for operation of the pneumatic compressor assembly by electrical means.

Alternatively, the gas pressure may be regulated mechanically by an adjustable spring functioning as set point device.

The gas pressure adjusted for operation of the pneumatic compressor assembly ranges preferably between 2.5 to 5 bar, more preferably between 3 to 5 bar, most preferably between 3 to 4 bar.

The gas pressure reducing means comprises a depressurizing capability in a certain ratio for reducing the pressure of the vaporized liquid gas from the pressurized gas container to the gas pressure as needed for the operation of the pneumatic compressor assembly. The gas pressure reducing means is adapted to provide a depressurizing capability preferably within a ratio of 10:1 to 30:1, more preferably within 12:1 to 25:1, most preferably within 15:1 to 20:1.

During decompression of the gas by the gas pressure reducing means, adiabatic expansion of the gas may occur, thereby providing a cooling effect. Preferably, the gas pressure reducing means comprises a means for the retrieval of cold. Such means may be used for the cooling at an application site of the patient's body.

Preferably, such means for the retrieval of cold is provided by a serpentine coiled around the means for the retrieval of cold. Preferably, the serpentine is a flexible tube. A fluid within the serpentine such as e.g. O₂ may be supplied at low pressure to the application site. Alternatively, the means for the retrieval of cold may be provided by a hollow jacket.

In a further aspect of the invention there is provided an apparatus comprising a branch with at least one outlet for coupling with the pressurized gas container at least one cannula sized and shaped for introduction into the patient's nasopharynx, the patient's mouth, the patient's trachea or the patient's stomach and/or intestine and at least one outlet for coupling a pneumatic compressor assembly with the pressurized gas container. Preferably, the at least one cannula is from an essentially rod-like shape and can be functionally connected to the pressurized gas container either directly or by means of a flexible tube.

Preferably, the cannula is sized and shaped for introduction into the interior of blood vessels, in particular the vena cava. More preferably, the cannula is adapted as an intravascular expandable balloon and provided with a cooling contact surface. Even more preferably, the expandable balloon is designed to have a star-like shape after inflation which advantageously increases the cooling contact surface.

Preferably, a gas pressure reducing means, in particular a gas pressure regulator, is provided between the pressurized gas container and the cannula allowing the adjustment of the working pressure. If gas from the same source is used for different applications, a separate pressure reducing means may be provided for each application. Such separate pressure reducing means may have different reduction rates.

While such an arrangement is conceivable for any source of pressurized gas, it is considered advantageous to use a pressurized gas allowing the operation of a pneumatic compressor assembly combined with the usage of such cannula.

In a still further embodiment, the pressurized gas container and the at least one cannula are adapted to provide for adiabatic expansion of the gas or mixture of gases, in particular as shown in EP 11191745.6 (incorporated herein by reference).

Such an apparatus is effective at cooling the brain or other internal areas or organs of a patient, particularly in case of cardiac arrest, acute myocardial infarction, ischemic stroke traumatic injury or neurogenic fever.

Thus, gases such as carbon dioxide allowing a combination of operating a pneumatic compressor assembly and cooling are most preferred. However, other gases effective substantially for the former or latter are advantageous as well.

In a further aspect of the invention there is provided an apparatus comprising a pressurized gas container and an inflatable bag, whereby the inflatable bag comprises a substantially closed sheath adapted to form an outer cooling contact surface upon inflation, and whereby the pressurized gas container and the inflatable bag are adapted to provide a cooling effect in the bag upon release of gas, e.g. by adiabatic expansion of the gas or mixture of gases.

Such inflatable bag with an outer cooling contact surface has the advantage that upon adiabatic expansion external cooling of the patient's body is achieved while the chest of the patient is still available e.g. for applying chest compressions.

WO 2006/000006 A2 proposes external cooling of a patient by means of an inflatable bag and a pressurized gas container storing liquid air, wherein, however, the inflatable bag fully envelopes the patient resulting in a direct contact between vaporized liquid air and the patient.

Preferably, the inflatable bag is adapted to form a tub upon inflation to surround substantially the patient on the rear and lateral side leaving the head and arms outside. Such a form of the inflatable bag has the advantage that the arms are available for intravenous liquid administration. More preferably, the inflatable bag is formed as sleeveless vest to be wrapped around the patient.

More preferably, a pressurized gas container and the inflatable bag are functionally connected either directly or by means of a flexible tube. Even more preferably, the pressurized gas container and the inflatable bag are adapted to provide for adiabatic expansion of the gas or mixture of gases stored in liquid form.

Still more preferably, at least one tube may be arranged within the bag, in particular at the walls of the bag, and the at least one tube is inflatable upon release of gas from the pressurized gas container. Preferably, the tube comprises at least one opening which is adapted to enable an adiabatic expansion of the gas when exiting the tube through the opening thereby providing a cooling effect.

Cooling of the bag may be achieved upon release of the gas, e.g. CO₂, into the bag and / or such tube within the bag comprising at least one opening adapted to enable adiabatic expansion of the gas. The bag may be inflated by pressurized gas from the pressurized gas container and / or additionally inflated by a pressurized gas from an additional gas source.

Preferably, an additional gas pressure reducing means, in particular a gas pressure regulator, is provided between the pressurized gas container and the inflatable bag and / or between the additional gas source and the inflatable bag allowing the adjustment of the working pressure.

According to a further preferred embodiment the branch comprises a switch valve for switching to at least one outlet for the pneumatic compressor assembly and to at least one outlet for the at least one cannula.

Preferably, the switch valve is provided with an opening and closing mechanism for alternatively opening the outlets.

Preferably, the switch valve is designed to allow opening simultaneously or alternatively of the at least one outlet for the pneumatic compressor assembly and the at least one outlet for the at least one cannula.

A switch valve advantageously allows changing between the operation modes of applying chest compressions during cardiopulmonary resuscitation (CPR) or cooling the brain or other internal areas or organs of a patient. Alternatively or additionally, a pressure reducing means, e.g. a gas pressure regulator or a pressure reducing valve, may be coupled to at least one outlet of the switch valve.

According to a preferred embodiment, the apparatus comprises a sensor for sensing compressions applied to the chest of the patient. More preferably the apparatus comprises a sensor for sensing the actuation of compressions by the actuator.

Sensing of the actuation of compression allows processing of the measured values by a control.

In a preferred embodiment, the apparatus comprises a control adapted to control and / or regulate the pressure and / or the frequency of compression to the chest of the patient, e.g. by controlling and / or regulating the application of pressurized gas to the actuator.

Preferably, the control is adapted to control and / or regulate the gas pressure reducing means.

Preferably, the apparatus comprises means for generating pulses of pressurized gas applied to the actuator between the pressurized gas container and the pneumatic compressor assembly, defining by means of such means for generating pulses the frequency of compression pulses to the chest of the patient.

Preferably, such means for generating pulses are realized by a valve. By opening and closing of a valve pulses of pressurized gas are generated. More preferably, opening and closing of such valve may be regulated mechanically in a manner known in the art without an active control means.

Alternatively, the control is adapted to control and / or regulate such means for generating pulses of pressurized gas. The control may be adapted to control and / or regulate opening and closing of such valve.

Alternatively, such means for generating pulses of pressurized gas may be a pulse generator.

Preferably, such pulses are generated by the pressure reducing valve by interrupting the passage for the gas flow.

Such pulses of pressurized gas mimic the applied compressions to the chest of a patient during cardiopulmonary resuscitation.

In a further preferred embodiment, the apparatus comprises a defibrillator. Preferably, the defibrillator comprises at least a component adapted to deliver pulses close to 1000 volts to the thorax of a patient with the aid of electrode pads.

The purpose of applying compressions to the chest of a patient during cardiopulmonary resuscitation (CPR) is to restore partial flow of oxygenated blood to the brain and heart. CPR alone is unlikely to restart the heart in case of ventricular fibrillation. Administration of one or several electric shocks to the patient's heart by a defibrillator is usually needed in order to restore a heart rhythm. Thus, a combined use with a defibrillator is advantageous.

In a further aspect of the invention there is provided an apparatus adapted to send a signal based on the actuation of compressions to the chest of a patient by an actuator to the defibrillator. Preferably, the defibrillator is coupled to the pneumatic compressor assembly, i.e. positioned around the actuator or contained in the housing of the pneumatic compressor assembly.

Within such arrangement, means for pCO₂ measurement may advantageously be added.

Preferably, the defibrillator includes or is coupled with an electrocardiogram measurement unit.

Preferably, the electrocardiogram measurement unit is adapted to perform electrocardiogram analyses (ECG analyses).

More preferably, the electrocardiogram measurement unit is adapted to perform pseudo electrocardiogram analyses (psECG analyses). A psECG analysis is defined as an ECG signal analysis recorded from the defibrillator pads not involving any measuring sites at the arms or legs.

Defibrillation should only be made for certain shockable heart rhythms such as ventricular fibrillation. Presence of such rhythms can be diagnosed by a pseudo electrocardiogram (psECG) measurement. The defibrillator may be adapted to give permission to deliver a shock and / or to deliver a shock automatically based on the psECG measurement.

A shockable heart rhythm is defined as a psECG analysis output that advises the use of a defibrillator shock or a sequence of defibrillator shocks to restore heart functionality.

The simultaneous operation of a device applying chest compressions to the patient during psECG measurement may cause undesired artifact signals in the psECG generated by the chest compression on the thorax of the patient. In particular, such artifact signal making the psECG analysis output difficult to read is caused by slight displacement of the electrode pads of the defibrillator during chest compression. Such artifact signals may lead to a wrong diagnosis.

Preferably, a sensor for sensing compressions by an actuator can be used to detect artifact signals.

According to a preferred embodiment, the actuator of the pneumatic compressor assembly comprises at least one cylinder and at least one piston sliding within the cylinder.

Preferably, the artifact signal detection is based on the displacement of electrodes during the piston stroke.

More preferably, the sensor for sensing the actuation of compressions is a sensor which measures the piston stroke.

In particular, such sensor which measures the piston stroke is adapted to measure the position of the piston within the cylinder of the actuator and to determine movement of the piston.

Preferably, such sensor which measures the piston stroke is a sensor using the Hall effect, i.e. a Hall effect sensor. Such Hall effect sensor may be arranged at the cylinder of the actuator whereby the Hall effect sensor detects the position of a magnet at the piston. Alternatively, such sensor which measures the piston stroke is based on piezoelectric technology.

Alternatively, the effectiveness of chest compression by actuation of compressions may be made by palpation of the femoral pulse and / or by using a blood pressure measurement device.

There is no displacement of the electrode pads of the defibrillator in absence of any chest compression, i.e. when the piston is standing still. When the piston moves within the cylinder during chest compression, displacement of the electrode pads occurs generating the artifact signal.

Alternatively, the artifact signal caused by the chest compression may be detected based on the energy input to the actuator of the pneumatic compressor assembly.

According to a further preferred embodiment, the defibrillator comprises an ECG measurement unit adapted to analyze ECG signals influenced by the chest compression. Preferably, the defibrillator is adapted to use a specific analysis algorithm adapted for diagnosing the presence of a shockable rhythm during CPR if the chest compression is detected by said sensor.

The chest compression pulses from the actuator of the pneumatic compressor assembly cause artifact signals regularly, i.e. in substantially constant time intervals. Preferably, such analysis algorithm for diagnosing the presence of a shockable rhythm during CPR is able to detect shockable heart rhythms e.g. based on the regularity of the generated artifacts. Preferably, the defibrillator comprises signal processing system, in particular a signal processing system, processing the incoming signals continuously with the aid of the analysis algorithm.

Such an analysis algorithm compensating ECG signals made on the chest compressions during CPR for diagnosing presence and time of shockable rhythms has been described in US 6,287,328. A further way of minimizing signal disturbances during CPR is disclosed in US 6,807,442.

Algorithms for analyzing artifact signals based on the compressions to the chest are usually performed on several ECG tests for diagnosing artifact signals. The direct measurement of the actuation of compressions by the actuator during CPR, preferably by a sensor, is easier and less error-prone than measurement on the patient and allows the operation of cardiovascular resuscitation and defibrillation in one apparatus.

In a further preferred embodiment, the application member is provided with at least two electrode pads of the defibrillator. Preferably, the at least two electrode pads are adapted to perform psECG analyses.

Preferably, the electrode pads are arranged at the fixing member. The at least two electrode pads are adapted to send an ECG signal to the ECG signal detection unit.

Preferably, the at least two electrode pads are arranged at the fixing member and adapted for detecting and recording ECG signals and administering a defibrillation shock.

It is further preferred that the at least two electrode pads are designed to adhere at the patient's body. This reduces the displacement of the electrode pads of the defibrillator during chest compression.

By arranging the electrode pads at the fixing member, the electrode pads are advantageously positioned at a usual measurement location for psECG measurements on the left- and right-hand site of the sternum.

Preferably, a cable connection is provided between the defibrillator and the at least two electrode pads and between the defibrillator and the sensor. More preferably, the cable connections are joined to one single-stranded cable to simplify the handling.

In a further aspect of the invention there is provided an apparatus comprising a pressurized gas container, preferably storing a gas in liquid form, a turbine, a electric power generator and a defibrillator, wherein the turbine is actuated by the pressurized gas and adapted for powering the electric power generator, and wherein the electric power generator is adapted for energizing a defibrillator.

While such apparatus is conceivable for any use of the defibrillator, it is considered advantageous to use such apparatus for energizing the defibrillator in combination with the apparatus for assisting cardiopulmonary resuscitation by applying compressions to the chest of a patient as shown above.

Such arrangement has the advantage that the defibrillator can be energized without the need of any batteries while still being transportable. Such arrangement may also be used in addition to batteries as further power supply support.

Preferably, such apparatus may further be adapted for energizing a control, in particular the control within the apparatus for assisting cardiopulmonary resuscitation by applying compressions to the chest of a patient as shown above.

Preferably, the apparatus comprises a container containing an incompressible fluid such as water between the pressurized gas container and the turbine, wherein the turbine is a hydro turbine.

The pressurized gas from the pressurized gas container presses the incompressible fluid towards to the hydroelectric turbine.

Such an arrangement with an additional container has the advantage that the incompressible fluid increases the force acting on the hydraulic turbine.

One further aspect of the present invention is a method for assisting cardiopulmonary resuscitation by applying compressions to the chest of a patient to stimulate blood circulation. This method is particularly useful for a patient suffering from cardiovascular emergency. An apparatus for assisting cardiovascular resuscitation as previously described is provided. An application member for pressing against an application site of the patient is applied to the application site. A pneumatic compressor assembly of the apparatus is connected with a pressurized gas container by a connecting member which is adapted for the operation of the pneumatic compressor assembly with a gas or mixture of gas in liquid form.

According to a preferred embodiment, the apparatus is operated by gas or a mixture of gas stored in liquid form.

A further aspect of the invention is method for assisting cardiopulmonary resuscitation. An apparatus is provided comprising a pressurized gas container containing a gas or mixture of gas stored in liquid form. The pressurized gas container supplies pressurized gas for the operation of a pneumatic compressor assembly. By a branch, the pressurized gas container is coupled to at least one cannula sized and shaped for introduction into the patient's nasopharynx, the patient's mouth, the patient's trachea or the patient's stomach and/or intestine.

A still further aspect of the invention is a method for cooling a patient externally. A pressurized gas container and an inflatable bag is provided whereby the pressurized gas container and the inflatable bag are adapted to provide for adiabatic expansion of a gas or mixture of gas, whereby the pressurized gas container stores a gas or mixture of gas. The pressurized gas container supplies the inflatable bag with the pressurized gas.

A further aspect of the invention is a method for assisting cardiopulmonary resuscitation by applying compressions to the chest of a patient. In a first step, a pneumatic compressor assembly is provided including at least an actuator and an application for pressing against an application site of the patient. In a next step the actuation of the compressions by the actuator is sensed.

Preferably, the sensed actuation is sent as an artifact signal to a defibrillator.

More preferably, a pseudo ECG measurement unit of the defibrillator processes the artifact signal based on the actuation of compressions.

The invention will be further outlined in the following with reference to preferred embodiments with drawings, without being limited thereto.
- Figure 1:: shows a schematic drawing of a preferred embodiment of an apparatus according to the present invention
- Figure 2:: shows a schematic drawing of a pressurized gas container with a connecting member, a gas pressure regulator and a spiral tube of a preferred embodiment of an apparatus according to the present invention
- Figure 3:: shows a cross-sectional view through the pneumatic compressor assembly according to the present invention
- Figure 4:: shows a cross-sectional view through an inflatable bag connected to a pressurized gas container according to the present invention
- Figure 5:: shows a schematic drawing of a preferred embodiment of an apparatus according to the present invention with a turbine and a generator coupled to the pressurized gas container

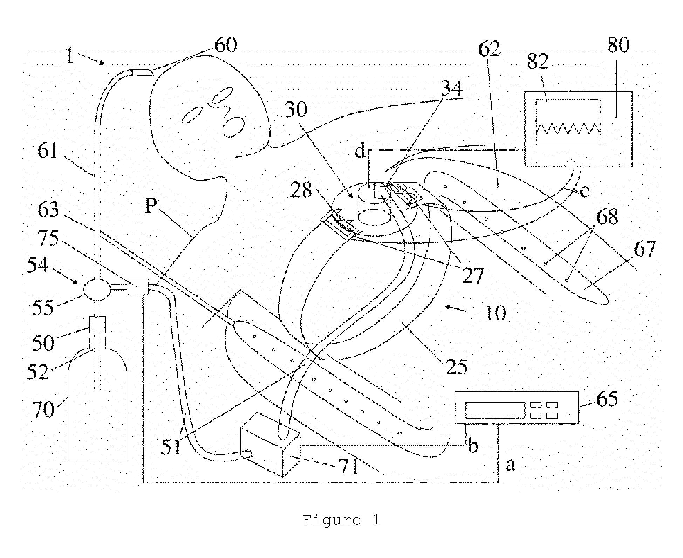

**Fig. 1** shows a schematic drawing of the parts of an apparatus 1 for assisting cardiopulmonary resuscitation. Fig.1 shows a pneumatic compressor assembly 10 which is arranged around the upper torso of a patient for pressing against the sternum of the patient's chest. Moreover, Fig.1 shows an actuator 30 arranged at a belt 25. The belt has a width of about 13 cm. The fixation of belt 25 is comprised by two eyelets 27 as end portions of the belt 25. Furthermore, Fig.1 shows four locking means 28 positioned at the actuator 30, in particular two pairs of locking means 28 positioned opposite to each other. The belt 25 is fixed to the chest of the patient in that the eyelets 27 are locked at the locking means 28. Furthermore, a pressing member (not shown in Fig.1) is arranged between the belt 25 and the sternum of the patient on the far side of the actuator 30. Two electrode pads (not shown in Fig.1) arranged at the belt 25 are connected to a defibrillator 80 via a cable connection e. The defibrillator 80 comprises an ECG measurement unit 82 for performing ECG tests and adapted to process artifact signals. A sensor (not shown in Fig.1) for sensing the actuation of compressions of the actuator 30 is connected with the defibrillator 80 by cable connection d. Cable connection d serves for sending a signal based on the actuation of compressions by the actuator 30 to the defibrillator 80. Cable connections d and e may be joined to one single-stranded cable for easier handling. The control 65 is connected to a membrane gas pressure regulator 75 by a cable connection a and a processing unit (not shown in Fig.1) of the control is adapted to control the adjustment of the pressure. The membrane gas pressure regulator 75 is provided by the company GCE Group (Gas Control Equipment). Fig.1 further shows a cable connection b between the control 65 and a pulse generator 71. Fig.1 further shows a pressurized gas container 70 which contains liquid carbon dioxide. The vapour pressure within the pressurized gas container is around 60 bar. The pressurized gas container is made of metal or composite material. Fig.1 further shows the flexible tube 51 extending into the interior of hollow space of the pressurized gas container 70. The pressurized gas container 70 has a quantity size of 3 l. The pressurized gas container 70 is required to be able to resist a maximum pressure of 120 bar. A connecting member 50 serves as connection to the pressurized gas container 70. A duct 52 is placed within the pressurized gas container 70 over the liquefied gas and functionally connected with the connecting member 50. A flexible tube 51 serves as connection of the connecting member 50 with the pneumatic compressor assembly 10 for supplying pressurized gas. The flexible tube 51 may be a conventional spiral tube made of e.g. polyurethane and/or polyamide. Furthermore, the flexible tube 51 serves for supplying pressurized gas to the membrane gas pressure regulator 75 and the pulse generator 71. The pulse generator 71 may be coupled to the actuator and not visible to the operator as a hidden mechanical device. Fig.1 further shows a branch 54 comprising a switch valve 55. The switch valve 55 allows switching of the gas supply between the flexible tube 51 and a flexible tube 61. Additionally or alternatively, a gas pressure regulator (not shown in Fig.1) may be coupled to an outlet of the switch valve 55. The flexible tube 61 allows supplying pressurized gas to a cannula 60 generating a cooling effect by adiabatic expansion. The flexible tube 61 comprises a branch 63 allowing the supply of pressurized gas to a tube 67 of an inflatable bag 62. The tube 67 is coiled along the patient-sided wall of the inflatable bag. Upon supply, pressurized gas expands adiabatically through openings 68 of the tube 67, thereby providing a cooling to the patient. The flexible tube 61 is reinforced, e.g. by a wire-mesh, in order to withstand the high pressure of around 60 bar as established in the pressurized gas container 70. The pressurized carbon dioxide expands when reaching the inflatable bag 62 and cools the patient's body down externally.

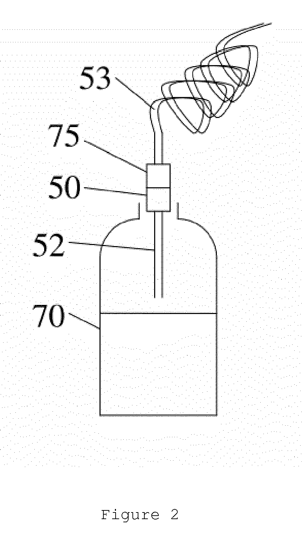

**Fig.2** shows a further preferred embodiment of an apparatus according to the present invention. Fig.1 shows a pressurized gas container 70 with a connecting member 50 serving as connection to the pressurized gas container 70. A duct 52 is placed within the pressurized gas container 70 over the liquefied gas and functionally connected with the connecting member 50. The membrane gas pressure regulator 75 is coupled to the connecting member 50. A spiral tube 53 serves as connection between the gas pressure regulator 75 and / or connecting member 50 and the pneumatic compressor assembly 10.

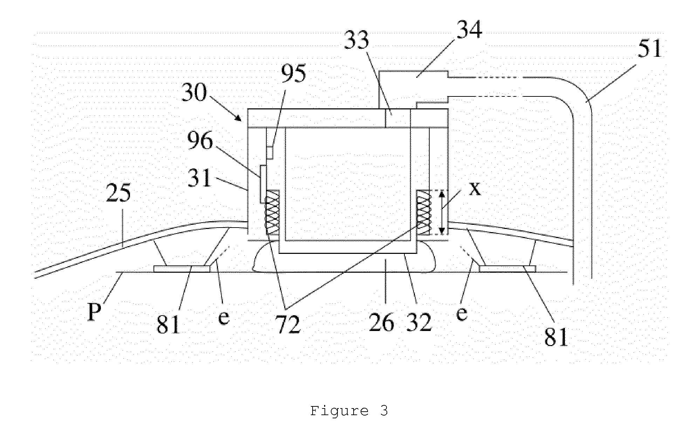

**Fig.3** shows a cross-sectional view through the pneumatic compressor assembly 10 (comparable to the "Weil™ Mini Chest Compressor" as known from the prior art) arranged at the sternum of the patient P. The application member 20 includes the actuator 30 and the belt 25. The actuator 30 includes a cylinder 31 and a piston 32 sliding within the cylinder 31. The cylinder 31 of the actuator 30 comprises an inlet 33 allowing pressurized gas to be supplied to the inlet 33 of the cylinder 31. Fig.3 schematically shows return springs 72 positioned between the piston 32 and the cylinder 31 returning the piston in the starting position. A connecting unit 34 is arranged at the actuator 30 which connects the flexible tube 51 to the actuator and provides an airtight connection the flexible tube 51 and the inlet 33. The pressurized gas presses downward against the piston 32 thereby compressing the patient's chest. The maximum downward travel x of the piston 32 is 5 cm. A sensor for measuring the piston stroke is arranged within the actuator 30. The sensor may be a Hall effect sensor 96 of the company "Motion Sensor Inc.. Such Hall effect sensor 96 is arranged at the cylinder 31 of the actuator 30 whereby the Hall effect sensor 96 detects the position of a magnet 95 arranged at the piston 32. A pressing member 26 is fixed at the side of the cylinder 31 facing the patient P functionally connecting the pressing member with the actuator 30. The pressing member 26 has a substantially round profile with a diameter of about 7 cm. The pressing member is comprised by a rigid plastic material. Two adhesive electrode pads 81 are mounted at the underside of the belt 25 contacting the breast of the patient P. The adhesive property of the electrode pads 81 reduces the displacement of the electrode pads of the defibrillator during chest compression. The adhesive electrode pads have a substantially rectangular profile with a breadth of 10 cm and a length of 12 cm. The adhesive electrode pads 81 are adapted for administering defibrillation shocks and for detecting ECG signals.

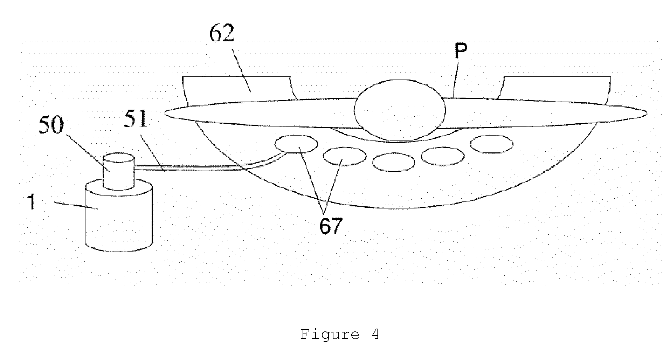

**Fig.4** shows a cross-sectional view through an inflatable bag 62 which is connected to a pressurized gas container 1 by a flexible tube 51 and a connecting member 50. The inflatable bag 62 is shaped and sized to surround the patient P on the rear and lateral side for effective external cooling. The tube 67 of the inflatable bag 62 is arranged along the patient-sided wall of the inflatable bag 62 for efficient external cooling of the patient.

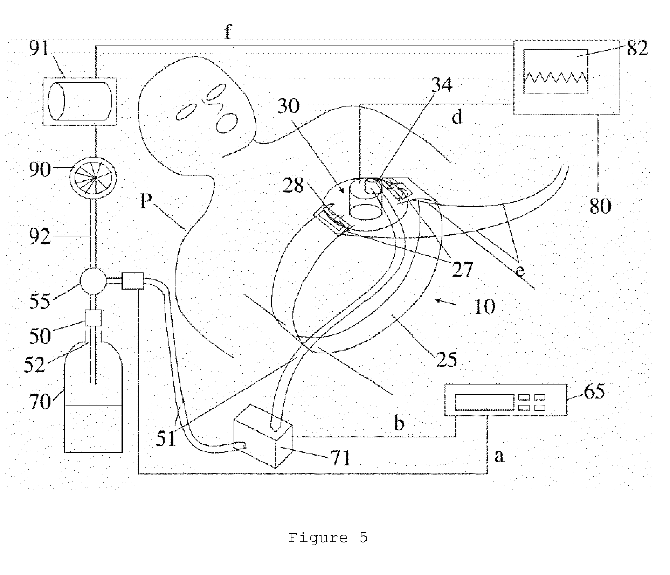

**Fig. 5** shows a schematic drawing of a preferred embodiment of an apparatus according to the present invention comprising the pneumatic compressor assembly 10 and the pressurized gas container 70 as shown in Fig.1. Fig.5 further shows a turbine 90 functionally coupled and powering to a electrical power generator 91. The turbine is functionally coupled and actuated with the pressurized gas container 70 by a flexible tube 92. The defibrillator 80 is energized through cable connection f by the generated energy of the electric power generator 91.

## Claims

1. Apparatus (1) for assisting cardiopulmonary resuscitation by applying compressions to the chest of a patient comprising a pneumatic compressor assembly (10) including at least an actuator (30) and an application member (20) for pressing against an application site of the patient,
wherein the pneumatic compressor assembly (10) is connected or connectable to a pressurized gas container (70) by a connecting member (50),
**characterized in that** the connecting member (50) is adapted for the operation of the pneumatic compressor assembly (10) with a gas or a mixture of gas stored in the container (70) in liquid form.

2. The apparatus (1) according to claim 1, **characterized in that** the apparatus (1) includes the pressurized gas container (70) which contains a gas or a mixture of gas stored in liquid form.

3. The apparatus (1) according to claim 1 or 2, **characterized in that** the apparatus (1) comprises a gas pressure reducing means between the pressurized gas container (70) and the pneumatic compressor assembly (10), in particular a gas pressure reducing means adapted to provide a depressurizing capability preferably within a ratio of 10:1 to 30:1, more preferably within 12:1 to 25:1, most preferably within 15:1 to 20:1.

4. An apparatus (1) preferably according to one of claims 1 to 3, wherein the apparatus (1) comprises a pressurized gas container (70) containing a gas or mixture of gas stored in liquid form, **characterized in that** the apparatus (1) comprises a branch (54) with
- at least one outlet (45) for coupling the pressurized fluid container with at least one cannula (60) sized and shaped for introduction into the patient's nasopharynx, the patient's mouth or the patient's trachea and
- at least one outlet for coupling the pressurized gas container (70) with a pneumatic compressor assembly (10) for applying compressions to the chest of a patient.

5. The apparatus (1) according to claim 4, **characterized in that** the pressurized gas container (70) and the at least one cannula (60) are adapted to provide for adiabatic expansion of the gas or mixture of gases stored in liquid form.

6. An apparatus (1) preferably according to one of claims 1 to 5, wherein the apparatus comprises a pressurized gas container (70) and an inflatable bag (62), wherein the inflatable bag comprises a substantially closed sheath adapted to form an outer cooling contact surface upon inflation, and wherein the pressurized gas container (70) and the inflatable bag (62) are adapted for cooling a patient externally, in particular by means of adiabatic expansion of a gas or mixture of gases stored in the container (70) in liquid form.

7. The apparatus (1) according to one of claims 4 to 6, **characterized in that** the branch (54) comprises a switch valve (55) for switching to at least one outlet for the pneumatic compressor assembly (10) and to at least one outlet for the at least one cannula (60).

8. An apparatus (1) preferably according to one of claims 1 to 7, wherein the apparatus (1) comprises an actuator (30) and an application member (20) for pressing against an application site of a patient, **characterized in that** the apparatus (1) comprises a sensor (40) for sensing the actuation of compressions by the actuator (30).

9. The apparatus (1) according to claim 8, **characterized in that** the sensor (40) for sensing the actuation of compressions by the actuator (30) is adapted to measure the piston stroke.

10. The apparatus (1) according to claim 9, **characterized in that** the sensor (40) is a Hall effect sensor and / or a sensor based on piezoelectric technology.

11. The apparatus (1) according to one of claims 1 to 10, **characterized in that** the apparatus (1) comprises a control (65) adapted to control and / or regulate the application of pressurized gas to the actuator (30).

12. The apparatus (1) according to one of claims 1 to 11, **characterized in that** the apparatus (1) comprises a defibrillator (80).

13. The apparatus (1) according to claim 12, **characterized in that** the apparatus (1) is adapted to send an artefact signal based on the sensed actuation of compressions by the actuator (30) to the defibrillator (80).

14. The apparatus (1) according to one of claims 8 to 13, **characterized in that** the actuator (30) of the pneumatic compressor assembly (10) comprises at least one cylinder (31) and at least one piston (32) sliding within the cylinder (31) and that the signal based on the actuation of compressions sent to the defibrillator (80) is based on the piston stroke.

15. The apparatus (1) according to one of claims 10 to 14, **characterized in that** the defibrillator (80) comprises an ECG measurement unit (82) and that the ECG measurement unit (82) is adapted to process artifact signals based on the actuation of compressions.

16. The apparatus according to one of claims 10 to 15, **characterized in that** the application member (20) is provided with at least two electrode pads (81) of the defibrillator (80).

17. An apparatus preferably according to one of claims 1 to 16, comprising a pressurized gas container (70), preferably storing a gas in liquid form, a turbine (90), a electrical power generator (91) and a defibrillator (80), wherein the turbine is actuated by the pressurized gas and adapted for powering the electric power generator (91), and wherein the electric power generator (91) is adapted for energizing the defibrillator (80)

18. Method for assisting cardiopulmonary resuscitation by applying compressions to the chest of a patient comprising the steps of:
a) applying an application member (20) of an apparatus (1) according to one of claims 1 to 15 to the application site of the patient
b) operating a pneumatic compressor assembly (10) of said apparatus (1) with a pressurized gas supplied from a pressurized gas container (70) storing a gas or a mixture of gas in liquid form.

19. Method for assisting cardiopulmonary resuscitation according to claim 18, **characterized in that** the apparatus (1) is connected with a pressurized gas container (70) containing gas or a mixture of gas in liquid form.

20. A method for assisting cardiopulmonary resuscitation preferably according to claim 17 or 19 comprising the steps of:
a) providing an apparatus comprising a pressurized gas container (70) containing a gas or mixture of gas stored in liquid form supplying pressurized gas for operating a pneumatic compressor assembly (10)
b) coupling the pressurized gas container (70) by a branch with at least one cannula (60) sized and shaped for introduction into the patient's nasopharynx, the patient's mouth, the patient's trachea or the patient's stomach and/ or intestine.

21. A method preferably according to one of claims 17 to 20 comprising the steps of:
a) providing a pressurized gas container (70) and an inflatable bag (62) adapted to provide for adiabatic expansion of a gas or mixture of gas
b) supplying the gas from the pressurized gas container (70) storing a gas or mixture of gas in liquid form to the inflate able bag (62).

22. A method preferably according to one of claims 17 to 21 for assisting cardiopulmonary resuscitation by applying compressions to the chest of a patient comprising the steps of:
a) providing a pneumatic compressor assembly (10) including at least an actuator (30) and an application member (20) for pressing against an application site of the patient according to one of claims 1 to 16
b) sensing the actuation of compressions by the actuator (30)

23. Method according to claim 22, **characterized in that** an artefact signal based on the sensed actuation of compressions by the actuator (30) is sent to a defibrillator (80).

24. Method according to claim 22, **characterized in that** an ECG measurement unit (82), in particular a pseudo ECG measurement unit, of the defibrillator (80) processes the artefact signal based on the actuation of compressions.
